# EUROPÄISCHE PATENTSCHRIFT

(11) **EP 2 667 830 B1**
(45) Veröffentlichungstag und Bekanntmachung des Hinweises auf die Patenterteilung: **06.04.2016**
(21) Anmeldenummer: 12701868.7
(22) Anmeldetag: 25.01.2012
(51) Int. Cl.: A61B 17/221, A61B 17/3207, A61F 2/915

(54) **MEDIZINISCHE VORRICHTUNG MIT EINER GITTERSTRUKTUR UND EIN BEHANDLUNGSYSTEM MIT EINER DERARTIGEN GITTERSTRUKTUR**
MEDICAL DEVICE HAVING A LATTICE STRUCTURE AND TREATMENT SYSTEM HAVING SUCH A LATTICE STRUCTURE
DISPOSITIF MÉDICAL AYANT UNE STRUCTURE DE TREILLIS ET UN SYSTÈME DE TRAITEMENT À L'AIDE D'UNE TELLE STRUCTURE DE TREILLIS

(30) Priorität: 25.01.2011 DE 102011009372
(43) Veröffentlichungstag der Anmeldung: 04.12.2013
(73) Patentinhaber: Acandis GmbH & Co. KG, 76327 Pfinztal (DE)
(72) Erfinder: CATTANEO, Giorgio, 76199 Karlsruhe (DE)
(74) Vertreter: Kilchert, Jochen
(86) Internationale Anmeldenummer: PCT/EP2012/051118
(87) Internationale Veröffentlichungsnummer: WO 2012/101159

(56) Entgegenhaltungen:
- EP-A1- 0 566 807
- WO-A1-2004/008991
- WO-A2-03/037398
- US-A1- 2004 102 834

## Beschreibung

Die Erfindung betrifft eine medizinische Vorrichtung mit einer komprimierbaren und expandierbaren, kreiszylinderförmigen Gitterstruktur, die Umfangssegmente aus geschlossenen Zellen umfasst.

Das technische Gebiet der Erfindung umfasst insbesondere stentähnliche Systeme bzw. Vorrichtungen zur Behandlung von Erkrankungen des Herz-Kreislaufsystems.

Die Druckschrift EP 0 566 807 A1 beschreibt einen Stent, der mehrere, sich parallel zur Längsachse des Stents erstreckende Stege aufweist. Die längsveriaufenden Stege sind durch querverlaufende Stege miteinander verbunden, so dass sich insgesamt parallelögrammartige Zellen bilden. Des Weiteren zählen dazu beispielsweise Vorrichtungen zum Entfernen von Blutgerinnseln, insbesondere Thrombectomie-Devices.

Aus der Praxis sind Thrombectomie-Devices bekannt, die eine korbartige Gitterstruktur umfassen. Die Gitterstruktur ist in radialer Richtung expandierbar und komprimierbar und wird über einen Zuführkatheter an den Behandlungsort gebracht. Dabei liegt die Gitterstruktur im Zuführkatheter im komprimierten Zustand vor. Durch Entlassen aus dem Zuführkatheter am Behandlungsort weitet sich die korbartige Gitterstruktur auf bzw. expandiert. Innerhalb eines Blutgefäßes weist die Gitterstruktur bedingt durch die Expansion einen größeren Querschnittsdurchmesser als innerhalb des Zuführkatheters, also im komprimierten Zustand, auf.

Die Gitterstruktur ist durch Stege gebildet, die Zellen begrenzen. Beim Übergang der Gitterstruktur vom radial komprimierten Zustand in den radial expandierten Zustand bewegen sich die Stege der Gitterstruktur ausschließlich in radialer Richtung. Ausgehend von einer Längsachse der Gitterstruktur entfernen sich die Stege der Gitterstruktur ausschließlich in radialer Richtung voneinander. Umgekehrt erfolgt bei der Komprimierung der Gitterstruktur eine radiale Bewegung der Stege in Richtung der Längsachse.

Am Behandlungsort, also beispielsweise im Bereich eines Thrombus, wird die Gitterstruktur bekannter Vorrichtungen expandiert, so dass sich die Stege im Wesentlichen geradlinig radial in den Thrombus bzw. das Blutgerinnsel einschneiden. Dadurch wird die Gitterstruktur und dem Blutgerinnsel verbunden.

Um das mit der Gitterstruktur verbundene Blutgerinnsel aus dem Blutgefäß zu entfernen, wird die Gitterstruktur bzw. allgemein die bekannte Vorrichtung aus dem Blutgefäß gezogen, wobei die Gitterstruktur auch Blutgefäße passiert, die einen größeren Querschnittsdurchmesser aufweisen als am Behandlungsort. Dabei besteht die Gefahr, dass sich das im Wesentlichen reibschlüssig mit den Stegen der Gitterstruktur verbundene Blutgerinnsel in radialer Richtung ablöst und durch die Blutströmung im Gefäß mitgerissen wird. Somit kann das Blutgerinnsel oder zumindest Teile des Blutgerinnsels zu einem erneuten Verschluss eines Blutgefäßes führen.

Bekannte Vorrichtungen werden auch dazu eingesetzt, Blutgerinnsel leichter von der Gefäßwand zu lösen. Dazu wird die expandierte Gitterstruktur beispielsweise von einem proximalen Ende des Blutgerinnsels entlang der Gefäßwand in das Blutgerinnsel geschoben, wobei die Gitterstruktur gleichzeitig manuell rotiert wird. Das bedeutet, dass der Anwender versucht, durch Drehung am proximalen Ende eines Führungsdrahts eine Rotation der Gitterstruktur, die am distalen Ende des Führungsdrahts angeordnet ist, zu bewirken. Dies gestaltet sich in der Praxis als äußerst schwierig, da einerseits die Umfangsfläche eines dünnen Führungsdrahts zu klein ist, um ausreichend Reibungskräfte zwischen den Fingern des Anwenders und dem Führungsdraht aufzubringen und den Führungsdraht zu drehen. Ferner bewirkt eine Drehung des proximalen Endes des Führungsdrahts zunächst eine Torsion des filigranen Führungsdrahts, so dass eine Rotation der am distalen Ende des Führungsdrahts angeordneten Gitterstruktur entweder überhaupt nicht oder zeitlich stark verzögert eintritt. Die Rotation der Gitterstruktur ist daher bei den bekannten Vorrichtungen schwer steuerbar.

Die Aufgabe der Erfindung besteht darin, eine medizinische Vorrichtung mit einer komprimierbaren und expandierbaren, kreiszylinderförmigen Gitterstruktur anzugeben, die eine verbesserte Verankerung in einem Blutgerinnsel ermöglicht, einfach steuerbar ist und eine gute Handhabbarkeit aufweist. Ferner besteht die Aufgabe der Erfindung darin, ein Behandlungssystem mit einer derartigen Vorrichtung anzugeben.

Erfindungsgemäß wird diese Aufgabe im Hinblick auf die medizinische Vorrichtung durch den Gegenstand des Patentanspruchs 1 und im Hinblick auf das Behandlungssystem durch den Gegenstand des Patentanspruchs 8 gelöst.

Die Erfindung beruht auf dem Gedanken, eine medizinische Vorrichtung mit einer komprimierbaren und expandierbaren, kreiszylinderförmigen Gitterstruktur anzugeben, die Umfangssegmente aus geschlossenen Zellen umfasst. Die Zellen sind durch jeweils vier Stege begrenzt, die an Verbindungsstellen miteinander gekoppelt sind und von denen jeweils zwei gegenüberliegend angeordnete Stege gleichartig ausgebildet sind und ein Stegpaar bilden. Die Stege eines ersten Stegpaares weisen zumindest abschnittsweise ein andere Form und/oder eine andere Stegbreite als die Stege eines zweiten Stegpaares auf derart, dass die Stege des ersten Stegpaares beim Übergang der Gitterstruktur vom expandierten Zustand in den komprimierten Zustand stärker verformt werden, als die Stege des zweiten Stegpaares. Jeweils ein Steg des ersten Stegpaares ist mit einem Steg des zweiten Stegpaares derart gekoppelt, dass zwei in Längsrichtung der Gitterstruktur gegenüberliegend angeordnete Verbindungsstellen beim Übergang der Gitterstruktur vom expandierten Zustand in den komprimierten Zustand in Umfangsrichtung der Gitterstruktur gegenläufig zueinander versetzt werden. Alle Zellen eines Umfangssegments sind gleichartig ausgebildet, so dass die gesamte Gitterstruktur beim Übergang vom expandierten Zustand in den komprimierten Zustand zumindest abschnittsweise tordiert.

Gemäß dem nebengeordneten Aspekt beruht die Erfindung auf dem Gedanken, ein Behandlungssystem mit der medizinischen Vorrichtung und einem Katheter anzugeben, wobei die medizinische Vorrichtung ein Führungselement, insbesondere einen Führungsdraht, umfasst, das mit einem axialen Ende der Gitterstruktur fest, insbesondere drehfest, verbunden und längsverschieblich in dem Katheter angeordnet ist.

Der Übergang der Gitterstruktur vom expandierten Zustand in den komprimierten Zustand, also bei der Komprimierung der Gitterstruktur, werden die einzelnen Stege der Gitterstruktur verformt. Diese Verformung wird bei der Expansion der Gitterstruktur, also beim Übergang der Gitterstruktur vom komprimierten Zustand in den expandierten Zustand, rückgängig gemacht. Alternativ kann vorgesehen sein, dass die Verformung der Stege bei der Expansion erfolgt und sich die Stege bei der Komprimierung wieder strecken. Insbesondere erfolgt die Verformung der einzelnen Stege in einem elastischen Bereich. Die Expansion bzw. Komprimierung der Gitterstruktur bezieht sich auf den Querschnittsdurchmesser. Bei der Komprimierung verringert sich der Querschnittsdurchmesser der Gitterstruktur, wogegen sich der Querschnittsdurchmesser bei der Expansion erhöht.

Bei der Erfindung ist vorgesehen, dass jeweils zwei gegenüberliegend angeordnete Stege einer Zelle ein Stegpaar bilden, dessen Stege sich bei der Zustandsänderung der Gitterstruktur, also bei der Komprimierung und/oder bei der Expansion, andersartig verformen, als die Stege eines weiteren Stegpaars derselben Zelle. Insbesondere weisen die Stege eines Stegpaars gleiche Eigenschaften auf. Konkret ist vorgesehen, dass die Stege eines ersten Stegpaars eine andere Form und/oder eine zumindest abschnittsweise andere Stegbreite als die Stege eines zweiten Stegpaars aufweisen. Dabei wird ein Stegpaar durch jeweils zwei Stege gebildet, die in der Zelle gegenüberliegend angeordnet sind, also nicht an einer Verbindungsstelle miteinander gekoppelt sind. Vielmehr sind an den Verbindungsstellen jeweils zwei Stege unterschiedlicher Stegpaare miteinander gekoppelt.

Durch die zumindest abschnittsweise unterschiedliche Form und/oder Stegbreite der Stege des ersten Stegpaars gegenüber den Stegen des zweiten Stegpaars wird erreicht, dass sich die in Längsrichtung gegenüberliegend angeordneten Verbindungsstellen einer Zelle bei der Zustandsänderung der Gitterstruktur, also beispielsweise beim Übergang vom komprimierten Zustand in den expandierten Zustand, gegenläufig zueinander versetzen. Mit anderen Worten erfolgt bei der Zustandsänderung der Gitterstruktur nicht nur eine Bewegung der in Längsrichtung gegenüberliegend angeordneten Verbindungsstellen parallel zur Längsachse der Gitterstruktur, also in Längsrichtung, sondern auch in Umfangsrichtung.

Die Gitterstruktur umfasst mehrere Umfangssegmente, die jeweils gleichartig ausgebildete Zellen umfassen. Die Umfangssegmente sind im Wesentlichen ringförmig ausgebildet und umfassen geschlossene Zellen, also Zellen die allseitig durch Stege begrenzt sind. Durch die zuvor erläuterten Form- und/oder Stegbreitenunterschiede zwischen den Stegen des ersten Stegpaars und des zweiten Stegpaars wird bewirkt, dass sich die Zellen eines Umfangssegments im Wesentlichen um einen in der Zelle angeordneten Drehpunkt drehen, wodurch sich insgesamt entlang der Gitterstruktur zumindest abschnittsweise eine Torsion einstellt.

Das bedeutet, dass sich die Stege der Gitterstruktur beim Übergang vom komprimierten Zustand in den expandierten Zustand, und umgekehrt, nicht nur geradlinig in radialer Richtung bewegen, sondern gleichzeitig eine Bewegung in Umfangsrichtung durchführen. Bei der Expansion der Gitterstruktur im Bereich eines Blutgerinnsels oder Thrombus wird so bewirkt, dass sich die Stege nicht nur geradlinig, sondern im Wesentlichen schraubenartig in das Blutgerinnsel bzw. den Thrombus einschneiden. Die Verankerung der Gitterstruktur bzw. allgemein der medizinischen Vorrichtung in einem Konkrement, insbesondere einem Blutgerinnsel oder einem Thrombus, wird somit verbessert.

Die Torsion der Gitterstruktur wird bereits durch die Expansion an sich bewirkt. Die Expansion erfolgt vorzugsweise selbsttätig, sobald ein durch einen Zuführkatheter aufgebrachter äußerer Zwang entfernt wird. Mit anderen Worten expandiert die Gitterstruktur vorzugsweise selbsttätig, wenn sie aus dem Zuführkatheter entlassen wird. Die Entlassung aus dem Zuführkatheter erfolgt durch eine längsaxiale Relativbewegung zwischen dem Zuführkatheter und der Gitterstruktur. Somit wird die Torsion der Gitterstruktur und die Umfangsbewegung der Stege der Gitterstruktur bereits durch eine translatorische Relativbewegung zwischen dem Zuführkatheter und der Gitterstruktur erreicht. Dies verbessert maßgeblich die Handhabbarkeit der medizinischen Vorrichtung. Insbesondere ist die Torsion der Gitterstruktur einfach steuerbar, da eine translatorische Relativbewegung über einen Führungsdraht besser übertragbar ist als eine Rotationsbewegung.

Gemäß einer bevorzugten Ausführungsform der erfindungsgemäßen Vorrichtung umfasst jede Zelle vier Verbindungsstellen, die im expandierten Zustand der Gitterstruktur eine rautenförmige Grundform der Zelle aufspannen. Dies gilt insbesondere für den vollständig expandierten Zustand, also den Herstellzustand der Gitterstruktur. Dabei ist vorgesehen, dass die gedachten Verbindungsgeraden zwischen den durch Stege verbundenen Verbindungsstellen einer Zelle gemeinsam eine Rautenform oder allgemein eine Parallelogrammform bilden. Vorzugsweise liegt zumindest in einem Zwischenzustand zwischen dem vollständig komprimierten Zustand und dem vollständig expandierten Zustand der Gitterstruktur eine Rautenform der Zelle vor. Durch die Zustandsänderung der Gitterstruktur und dem damit verbundenen gegenläufigen Versatz, der in Längsrichtung gegenüberliegend angeordneten Verbindungsstellen, verformt sich die einzelne Zelle derart, dass die Grundform in eine parallelogrammartige Form übergeht. Die rautenförmige Grundform der Zelle ist für die Torsion der Gitterstruktur besonders vorteilhaft.

In einer weiteren Ausgestaltung der erfindungsgemäßen medizinischen Vorrichtung ist vorgesehen, dass die Stege des ersten Stegpaares im Wesentlichen S-förmig und die Stege des zweiten Stegpaares im Wesentlichen geradlinig ausgebildet sind. Die S-förmig gekrümmten Stege verformen sich bei der Zustandsänderung der Gitterstruktur stärker als die geradlinig ausgebildeten Stege.

Alternativ oder zusätzlich können die Stege des ersten Stegpaares eine Stegbreite aufweisen, die kleiner als die Stegbreite der Stege des zweiten Stegpaares ist. Das erste Stegpaar kann sich also von dem zweiten Stegpaar durch die unterschiedliche Breite der Stege unterscheiden. Dabei sind die Stege eines einzigen Stegpaares gleichartig ausgebildet, weisen also die gleiche Stegbreite auf. Die Stegbreite kann sich auch nur abschnittsweise entlang der Stege unterscheiden. Das bedeutet, dass das erste Stegpaar Stege aufweisen kann, die jeweils einen Abschnitt umfassen, in dem die Stegbreite der Stege des ersten Stegpaares kleiner als sie Stegbreite der Stege des zweiten Stegpaares ist. Die Stege des ersten Stegpaares, die zumindest abschnittsweise eine kleinere Stegbreite als die Stege des zweiten Stegpaares aufweisen, weisen somit eine vergleichsweise höhere Verformbarkeit bei der Zustandsänderung der Gitterstruktur auf.

Die unterschiedliche Form und/oder die unterschiedliche Stegbreite zwischen den Stegen des ersten Stegpaars und den Stegen des zweiten Stegpaares kann in einer weiteren bevorzugten Ausführungsform durch Biegestellen dargestellt sein, die in den Stegen des ersten Stegpaares angeordnet sind. Die Stege des ersten Stegpaares können also jeweils eine Biegestelle aufweisen, an der die Stegbreite und/oder die Stegdicke des jeweiligen Stegs zumindest abschnittsweise reduziert oder erhöht ist. Alternativ oder zusätzlich kann die Biegestelle auch durch eine Formänderung des jeweiligen Stegs erreicht werden. Beispielsweise kann der Steg wenigstens einen Durchbruch und/oder ein Fenster aufweisen, das eine Biegestelle bildet. Insgesamt ist im Bereich der Biegestelle die Gesamtbreite und/oder Gesamtquerschnittsfläche des jeweiligen Stegs reduziert, so dass im Wesentlichen Sollbiegestellen bzw. Knickpunkte gebildet sind, in welchen der Steg vergleichsweise leicht verformbar ist. Durch die Biegestellen sind die Stege des ersten Stegpaares also verformbarer als die Stege des zweiten Stegpaares.

Die vorgenannten Konstruktionsmerkmale bewirken gemeinsam oder für sich genommen, dass die Stege des ersten Stegpaares bei der Zustandsänderung der Gitterstruktur, also beim Übergang vom expandierten Zustand in den komprimierten Zustand und umgekehrt, verformbarer als die Stege des zweiten Stegpaares sind. Dabei ist die Verformbarkeit der Stege eines einzigen Stegpaares untereinander gleich. Das bewirkt, dass sich bei der Zustandsänderung der Gitterstruktur ein gleichmäßiger Versatz zwischen den in Längsrichtung gegenüberliegend angeordneten Verbindungsstellen einstellt. Mit anderen Worten werden die in Längsrichtung gegenüberliegend angeordneten Verbindungsstellen ausgehend von einer Nullpunktlage um den gleichen Betrag in Umfangsrichtung der Gitterstruktur bewegt, wobei die Bewegung der beiden Verbindungsstellen gegenläufig erfolgt. Eine erste Verbindungsstelle der Zelle bewegt sich also um denselben Betrag auf dem Umfang der Gitterstruktur in Uhrzeigerrichtung, wie die in Längsrichtung gegenüberliegend angeordnete Verbindungsstelle sich in Gegenuhrzeigerrichtung bewegt.

Die Stege einer Zelle sind an den Verbindungsstellen einstückig verbunden. Auf diese Weise wird eine Relativbewegung der Stege untereinander unterbunden. Die Verformung der Stege beim Übergang vom komprimierten Zustand der Gitterstruktur in den expandierten Zustand und umgekehrt wird also durch eine flexible bzw. elastische Verbiegung bzw. Auslenkung der einzelnen Stege erreicht. Damit wird eine besonders hohe Stabilität der gesamten Gitterstruktur erreicht.

Bei einer weiteren bevorzugten Ausführungsform der erfindungsgemäßen medizinischen Vorrichtung umfassen die Umfangssegmente jeweils zwei Teilsegmente, die jeweils mäanderförmig angeordnete Stege aufweisen, wobei jeder zweite Steg eines Teilsegments gleichartig ausgebildet ist. Die Teilsegmente sind also durch mäanderförmig angeordnete Stege gebildet, wobei die Stege jeweils an Verbindungsstellen miteinander gekoppelt sind. Jeder zweite Steg des Teilsegments weist die selbe Form und/oder Stegbreite auf. Mit anderen Worten sind in einem Teilsegment unterschiedlich verformbare Stege alternierend mäanderförmig angeordnet. Die Herstellung der Vorrichtung ist somit vereinfacht.

Die Erfindung wird im Folgenden anhand von Ausführungsbeispielen unter Bezugnahme auf die beigefügten schematischen Zeichnungen näher erläutert. Darin zeigen
- Figur 1:: eine Draufsicht auf eine Zelle der Gitterstruktur der erfindungsgemäßen medizinischen Vorrichtung nach einem bevorzugten Ausführungsbeispiel im expandierten Zustand;
- Figur 2:: die Zelle gemäß Figur 1 im komprimierten Zustand;
- Figur 3:: einen Ausschnitt der Gitterstruktur mit mehreren Zellen gemäß Figur 1 im expandierten Zustand;
- Figur 4:: die Gitterstruktur gemäß Figur 3 im komprimierten Zustand;
- Figur 5:: eine Draufsicht auf eine Zelle der Gitterstruktur der erfindungsgemäßen medizinischen Vorrichtung nach einem weiteren bevorzugten Ausführungsbeispiel im expandierten Zustand, wobei sich die Stege der Stegpaare durch ihre Stegbreite unterscheiden;
- Figur 6:: die Zelle gemäß Figur 5 im komprimierten Zustand;
- Figur 7:: eine Draufsicht auf eine Zelle der Gitterstruktur der erfindungsgemäßen medizinischen Vorrichtung nach einem weiteren bevorzugten Ausführungsbeispiel im expandierten Zustand, wobei zwei Stege eines Stegpaars jeweils eine Biegestelle aufweisen; und
- Figur 8:: einen Querschnitt durch ein Blutgefäß mit einem Blutgerinnsel und der darin angeordneten Gitterstruktur der erfindungsgemäßen medizinischen Vorrichtung im Gebrauch.

Die nachfolgende Detailbeschreibung der medizinischen Vorrichtung bezieht sich auf die Gitterstruktur 10 der medizinischen Vorrichtung im Herstellzustand, also im vollständig expandierten Zustand der Gitterstruktur 10, soweit nichts anderes angegeben ist. Der Bezugspunkt für medizinische Richtungsangaben, insbesondere Richtungsangaben "proximal" und "distal" ist der Anwender der medizinischen Vorrichtung bzw. des Behandlungssystems. Proximal angeordnete Bauteile sind dem Anwender der Vorrichtung bzw. des Behandlungssystems also näher als distal angeordnete Bauteile.

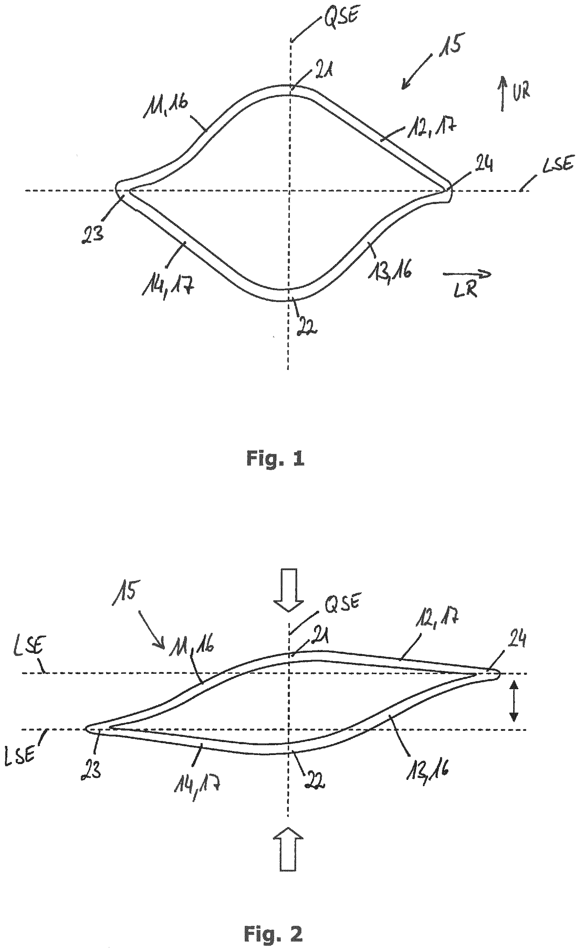

In Figur 1 ist eine freigeschnittene, geschlossene Zelle 15 einer Gitterstruktur 10 der erfindungsgemäßen medizinischen Vorrichtung nach einem bevorzugten Ausführungsbeispiel dargestellt. Die Zelle umfasst vier Stege 11, 12, 13, 14, die an Verbindungsstellen 21, 22, 23, 24 miteinander gekoppelt sind. Insbesondere sind die Stege 11, 12, 13, 14 einstückig an den Verbindungsstellen 21, 22, 23, 24 miteinander verbunden. Die miteinander gekoppelten Stege 11, 12, 13, 14 begrenzen somit die Zelle 15. Die Zelle 15 weist im Wesentlichen eine rautenförmige Grundform auf. Konkret bilden die Verbindungsstellen 21, 22, 23, 24 die Eckpunkte eine Raute, wobei sich die Stege 11, 12, 13, 14 im Wesentlichen entlang der Seitenlinien der Raute erstrecken und die Verbindungsstellen 21, 22, 23, 24 miteinander verbinden. Dabei folgen die Stege 11, 12, 13, 14 nicht streng den Verbindungslinien zwischen den Verbindungsstellen 21, 22, 23, 24, sondern können eine Form aufweisen, die vom geradlinigen Verlauf der Verbindungslinien abweicht. Die Grundform einer Raute bleibt jedoch erkennbar. Vorzugsweise weist die Zelle 15 zumindest in einem Zustand der Gitterstruktur 10, also in einem komprimierten Zustand oder einem expandierten Zustand oder einem Zwischenzustand, die rautenförmige Grundform auf. In wenigstens einem weiteren Zustand der Gitterstruktur 10 bildet die Zelle 15 vorzugsweise eine parallelogrammartige Grundform. Die parallelogrammartige Grundform unterscheidet sich von der rautenförmigen Grundform dadurch, dass die diagonalen Verbindungslinien zwischen jeweils zwei Verbindungsstellen 21, 22, 23, 24 bei der Rautenform orthogonal zueinander ausgerichtet sind. Bei der parallelogrammartigen Grundform weisen die diagonalen Verbindungslinien zwischen zwei gegenüberliegenden Verbindungsstellen 21, 22, 23, 24 zueinander einen Winkel auf, der von einem rechten Winkel abweicht. Mit anderen Worten ist bei der rautenförmigen Grundform vorgesehen, dass die in Längsrichtung LR der Gitterstruktur 10 gegenüberliegend angeordneten Verbindungsstellen 23, 24 in einer Längsschnittebene LSE der Gitterstruktur 10 angeordnet sind, in der auch die Langsachse der Gitterstruktur 10 verläuft. Die in Umfangsrichtung UR der Gitterstruktur 10 gegenüberliegend angeordneten Verbindungsstellen 21, 22, 23, 24 sind bei der rautenförmigen Grundform in einer Querschnittsebene QSE der Gitterstruktur 10 angeordnet, die orthogonal zur Längsachse bzw. zur Längsschnittebene LSE der Gitterstruktur 10 angeordnet ist. Bei der parallelogrammartigen Grundform sind die in Längsrichtung LR der Gitterstruktur 10 gegenüberliegend angeordneten Verbindungsstellen 23, 24 hingegen in Umfangsrichtung UR zueinander versetzt, so dass die diagonale Verbindungslinie zwischen in Längsrichtung gegenüberliegend angeordneten Verbindungsstellen 23, 24 die Querschnittsebene QSE unter einem Winkel schneidet.

Die Zelle 15 weist einen ersten Steg 11, einen zweiten Steg 12, einen dritten Steg 13 und einen vierten Steg 14 auf. Der erste Steg 11 erstreckt sich zwischen einer ersten Verbindungsstelle 21 und einer dritten Verbindungsstelle 23. Der zweite Steg 12 verbindet die erste Verbindungsstelle 21 mit einer vierten Verbindungsstelle 24. Der dritte Steg 13 ist mit dem zweiten Steg 12 durch die vierte Verbindungsstelle 24 und mit dem vierten Steg 14 durch eine zweite Verbindungsstelle 22 gekoppelt. Der vierte Steg 14 verbindet die zweite Verbindungsstelle 22 mit der dritten Verbindungsstelle 23. Die erste Verbindungsstelle 21 und die zweite Verbindungsstelle 22 sind in Umfangsrichtung UR der Gitterstruktur 10 gegenüberliegend angeordnet. Die dritte Verbindungsstelle 23 und die vierte Verbindungsstelle 24 sind in Längsrichtung LR der Gitterstruktur 10 gegenüberliegend angeordnet.

Der erste Steg 11 und der dritte Steg 13 sind in der Zelle 15 diagonal gegenüberliegend angeordnet und jeweils durch den zweiten Steg 12 und den vierten Steg 14 miteinander verbunden. Der erste Steg 11 und der dritte Steg 13 bilden gemeinsam ein erstes Stegpaar 16. Der zweite Stege 12 und der vierte Steg 14 sind bezogen auf die Zelle 15 diagonal gegenüberliegend angeordnet und bilden ein zweites Stegpaar 17.

Die Stegpaare 16, 17 weisen jeweils Stege 11, 12, 13, 14 auf, die gleichartig ausgebildet sind. Insbesondere weisen die Stege 11, 13 des ersten Stegpaars 16 im Wesentlichen die selbe Form und die selben Dimensionen, insbesondere betreffend Stegbreite und Stegdicke, auf. Dasselbe gilt für die Stege des zweiten Stegpaars, also den zweiten Steg 12 und den vierten Steg 14. Die Stege 11, 12, 13, 14 unterschiedlicher Stegpaare 16, 17 unterscheiden sich jedoch in ihrer Form und/oder Stegbreite. Insbesondere sind die Stege 11, 13 des ersten Stegpaars 16 gegenüber den Stegen 12, 14 des zweiten Stegpaars 17 derart andersartig geformt und/oder dimensioniert, dass die Stege 11, 13 des ersten Stegpaars 16 beim Übergang der Gitterstruktur 10 von einem radial expandierten Zustand in einen radial komprimierten Zustand und umgekehrt stärker verformbar als die Stege 12, 14 des zweiten Stegpaars 17 sind. Damit wird erreicht, dass sich die dritte Verbindungsstelle 23 und die vierte Verbindungsstelle 24 bei der Zustandsänderung der Gitterstruktur 10 in Umfangsrichtung UR der Gitterstruktur 10 gegenläufig bewegen, sich also versetzen. Insbesondere bei der Komprimierung der Gitterstruktur 10 werden die dritte Verbindungsstelle 23 und die vierte Verbindungsstelle 24 bzw. allgemein die in Längsrichtung LR der Gitterstruktur 10 gegenüberliegend angeordneten Verbindungsstellen 23, 24 aus der ursprünglichen Lage in der Längsschnittebene LSE gegenläufig ausgelenkt, so dass sich in Umfangsrichtung UR der Gitterstruktur 10 ein Abstand zwischen der dritten Verbindungsstelle 23 und der vierten Verbindungsstelle 24 einstellt, wie durch den Doppelpfeil in Figur 2 dargestellt.

Figur 2 zeigt die Zelle gemäß Figur 1 im komprimierten Zustand, wobei erkennbar ist, dass durch die höhere Verformbarkeit der Stege 11, 13 des ersten Stegpaares eine Auslenkung der dritten Verbindungsstelle 23 und der vierten Verbindungsstelle 24 erfolgt, so dass die Zelle 15 bei der Komprimierung der Gitterstruktur 10 von einer rautenförmigen Grundform in eine parallelogrammartige Grundform übergeht. Bei der Komprimierung der Gitterstruktur 10 nähern sich die erste Verbindungsstelle 21 und die zweite Verbindungsstelle 22 einander an, wie durch die Blockpfeile in Figur 2 symbolisiert. Gleichzeitig wird die Zelle 15 gestreckt. Das bedeutet, dass sich die dritte Verbindungsstelle 23 und die vierte Verbindungsstelle 24 in Längsrichtung LR der Gitterstruktur 10 voneinander entfernen. Dabei entfernen sich die dritte Verbindungsstelle 23 und die vierte Verbindungsstelle 24 auch in Umfangsrichtung UR der Gitterstruktur 10 voneinander, so dass im Wesentlichen von einer Drehung der Zelle 15 um einen in der Zelle angeordneten Drehmittelpunkt gesprochen werden kann. Da die Zelle 15 teil eines Umfangssegments 20 der Gitterstruktur 10 ist, das mehrere Zellen, insbesondere mehrere gleichartig ausgebildete Zellen 15, umfasst und einen geschlossenen Zellenring bildet, wird ein Versatz der in Umfangsrichtung gegenüberliegend angeordneten Verbindungsstellen 21, 22, also der ersten Verbindungsstelle 21 und der zweiten Verbindungsstelle 22, vermieden. Dies führt dazu, dass sich die Zustandsänderung der Gitterstruktur 10, also die Komprimierung oder die Expansion, nur auf einen Versatz zwischen den in Längsrichtung LR der Gitterstruktur 10 gegenüberliegend angeordneten Verbindungsstellen 23, 24 auswirkt.

Bei dem Ausführungsbeispiel gemäß Figuren 1-4 wird die erhöhte Verformbarkeit der Stege 11, 13 des ersten Stegpaars 16 durch die besondere Form des ersten Stegs 11 und des dritten Stegs 13 erreicht. Insbesondere sind der erste Steg 11 und der dritte Steg 13 im Wesentlichen S-förmig gekrümmt. Mit anderen Worten zeigen der erste Steg 11 und der dritte Steg 13 zwischen ihren jeweiligen Verbindungsstellen 21, 22, 23, 24 zu den Stegen 12, 14 des zweiten Stegpaares 17 einen S-förmigen Verlauf. Die Stege 12, 14 des zweiten Stegpaares 17 verlaufen zwischen Ihren jeweiligen Verbindungsstellen 21, 22, 23, 24 hingegen geradlinig. Daher weißt der zweite Steg 12 und der vierte Steg 14 eine geringere Verformbarkeit bzw. eine höhere Steifigkeit als der erste Steg 11 und der dritte Steg 13 auf. Bei der Zustandsänderung der Gitterstruktur 10 verformen sich der erste Steg 11 und der dritte Steg 13 daher stärker als der zweite Steg 12 und der vierte Steg 14. Die Stege 11, 13 des ersten Stegpaares 16 sind also im Allgemeinen biegsamer bzw. flexibler als die Steg 12, 14 des zweiten Stegpaares 17. Die Verformbarkeit oder Biegsamkeit bzw. Flexibilität des ersten Stegs 11 und des dritten Stegs 13, also der Stege des ersten Stegpaars 16 untereinander, ist im Wesentlichen gleich. Ebenso weisen der zweite Steg 12 und der vierte Steg 14, also die Stege 12, 14 des zweiten Stegpaars 17 untereinander, die gleiche Verformbarkeit oder Biegsamkeit bzw. Flexibilität auf.

Die medizinische Vorrichtung weist im Allgemeinen eine Gitterstruktur 10 auf, die eine Vielzahl von Zellen 15 umfasst. Insbesondere umfasst die Gitterstruktur 10 Umfangssegmente 20, die mehrere Zellen 15 aufweisen. Die Umfangssegmente 20 bilden jeweils einen Zellenring aus Zellen 15, der sich um die Längsachse der Gitterstruktur 10 erstreckt. Die Umfangssegmente 20 der Gitterstruktur 10 sind in Längsrichtung LR der Gitterstruktur 10 miteinander verbunden, so dass sich insgesamt eine geschlossene Gitterstruktur 10 bildet. Die Zellen 15 eines einzigen Umfangssegments 20 sind gleichartig ausgebildet. Dadurch ist sichergestellt, dass sich in jedem einzelnen Umfangssegment 20 derselbe Versatz der in Längsrichtung gegenüberliegend angeordneten Verbindungsstellen 23, 24 einstellt.

Die Gitterstruktur 10 kann allgemein einstückig ausgebildet sein. Insbesondere kann die Gitterstruktur 10 einstückig aus einem Vollmaterial durch Ausschneiden der Zellöffnungen hergestellt sein. Durch den Materialabtrag in den Zellen 15 werden dabei die Stege 11, 12, 13, 14 freigelegt. Vorzugsweise ist die Gitterstruktur 10 durch Laserschneiden hergestellt bzw. bildet eine lasergeschnittene Gitterstruktur 10. Zumindest abschnittsweise ist die Gitterstruktur 10 kreiszylinderförmig ausgebildet. Die Gitterstruktur 10 bildet also eine Wandungsebene, die sich kreiszylinderförmig um die Längsachse der Gitterstruktur 10 erstreckt. Auf diese Weise wird eine zumindest abschnittsweise röhrchenartige, insbesondere stentartige, Gitterstruktur 10 gebildet.

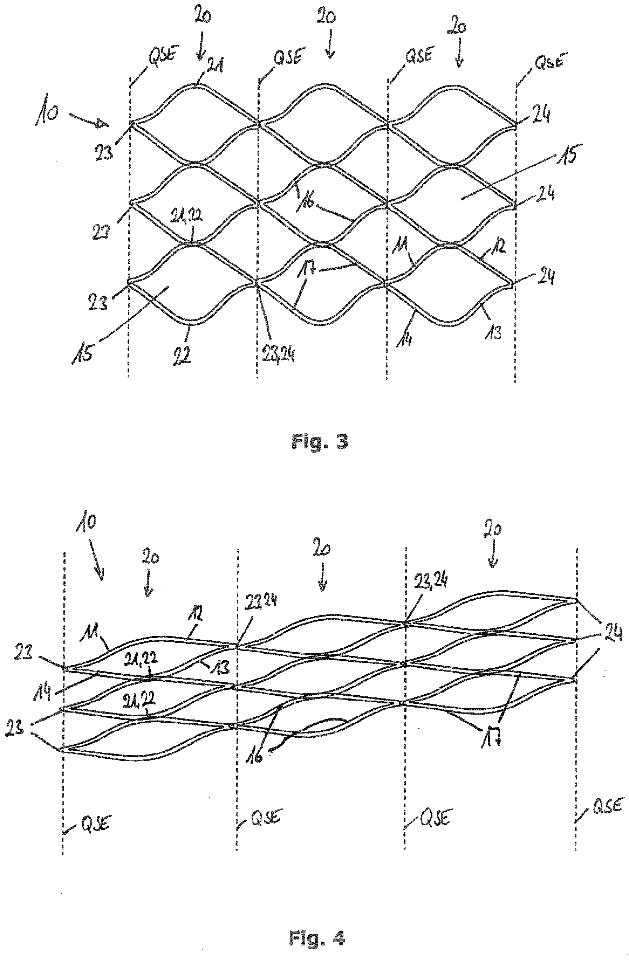

In Figur 3 ist zu erkennen, dass mehrere Umfangssegmente 20 mit gleichartig ausgebildeten Zellen 15 die Gitterstruktur 10 bilden können. Insbesondere zeigt Figur 3 einen Ausschnitt der Gitterstruktur 10, wobei drei Umfangssegmente 20 dargestellt sind, die jeweils Zellen 15 umfassten, wobei die Zellen 15 aller drei Umfangssegmente 20 gleichartig ausgebildet sind. Insbesondere weisen die Zellen 15 jeweils zwei Stegpaare 16, 17 auf, wobei das erste Stegpaar 16 S-förmig gekrümmte Stege 11, 13 und das zweite Stegpaar 17 geradlinig ausgebildete Stege 12, 14 aufweist. Figur 3 zeigt den expandierten Zustand der Gitterstruktur 10, wobei die Zellen 15 eine rautenförmige Grundform aufweisen. Durch die in der Zeichnungsebene vertikal verlaufenden gestrichelten Linien sind einerseits die Begrenzungen der Umfangssegmente 20 dargestellt. Andererseits zeigen die vertikal verlaufenden gestrichelten Linien die Lage einzelner Querschnittsebenen QSE an, in denen jeweils Verbindungsstellen 21, 22, 23, 24 der Stege 11, 12, 13, 14 angeordnet sind. Die Verbindungsstellen 21, 22, 23, 24 bewegen sich bei der Zustandsänderung der Gitterstruktur 10, also beispielsweise beim Übergang vom expandierten Zustand in den komprimierten Zustand, entlang der Querschnittsebenen QSE. Die in Längsrichtung gegenüberliegend angeordneten Verbindungsstellen 23, 24 der einzelnen Zellen 15 bewegen sich dabei gegenläufig in Umfangsrichtung UR der Gitterstruktur 10. Bei der Gitterstruktur gemäß Figur 3 erfolgt durch die Komprimierung ein Versatz der in Längsrichtung LR der Gitterstruktur 10 gegenüberliegend angeordnete Verbindungsstellen 23, 24, also der dritten und vierten Verbindungsstellen 23, 24 der Zellen 15. Da sich die in Längsrichtung gegenüberliegend angeordneten Verbindungsstellen 23, 24 aller Zellen 15 benachbarter Umfangssegmente 20 zueinander versetzen, erfolgt insgesamt eine Torsion der Gitterstruktur 10 beim Übergang vom radial expandierten Zustand, wie in Figur 3 dargestellt, in den radial komprimierten Zustand, wie in Figur 4 gezeigt. In Figur 4 ist gut erkennbar, dass durch den Versatz der in Längsrichtung gegenüberliegen angeordneten Verbindungsstellen 23, 24 der einzelnen Zellen 15 erreicht wird, dass sich die einzelnen Umfangssegmente 20 der Gitterstruktur 10 zueinander verdrehen. Allein durch die radiale Aufweitung bzw. Komprimierung der Gitterstruktur 10 wird also eine schraubenartige Torsionsbewegung der Gitterstruktur 10 bewirkt.

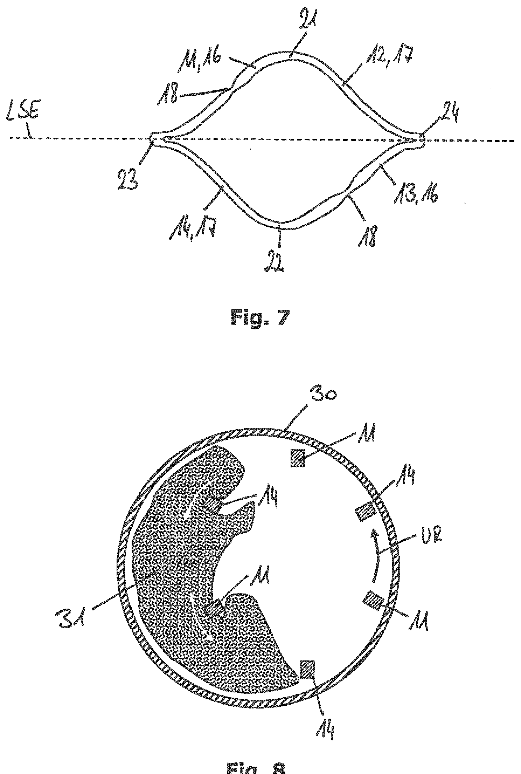

Im Gebrauch wird durch die Torsion der Gitterstruktur 10, die einerseits bei der Expansion und andererseits auch bei der Komprimierung erfolgt, erreicht, dass sich die Stege 11, 12, 13, 14 der Gitterstruktur 10 beispielsweise in ein Blutgerinnsel 31 schraubenartig einschneiden, wie in Figur 8 beispielhaft dargestellt. Figur 8 zeigt einen Querschnitt durch ein Blutgefäß 30, in dem Blutgerinnsel 31 angeordnet ist. Ferner ist ein Querschnitt durch die Gitterstruktur 10 schematisch dargestellt, wobei erkennbar ist, dass sich die Stege 11, 12, 13, 14 der Gitterstruktur 10 bei der Expansion der Gitterstruktur 10 nicht nur in radiale Richtung ausgehend von der Längsachse der Gitterstruktur 10 in das Blutgerinnsel 31 einschneiden, sondern sich auch in Umfangsrichtung UR der Gitterstruktur 10 in das Blutgerinnsel 31 eingreifen. Die Umfangsrichtung UR bzw. die in Umfangsrichtung gerichtete Bewegung der Stege 11, 12, 13, 14 ist durch Pfeile in Figur 8 dargestellt. Auf diese Weise werden im Blutgerinnsel 31 Hinterschnitte gebildet, die zu einer verbesserten Anhaftung des Blutgerinnsels 31 an der Gitterstruktur 10 beitragen.

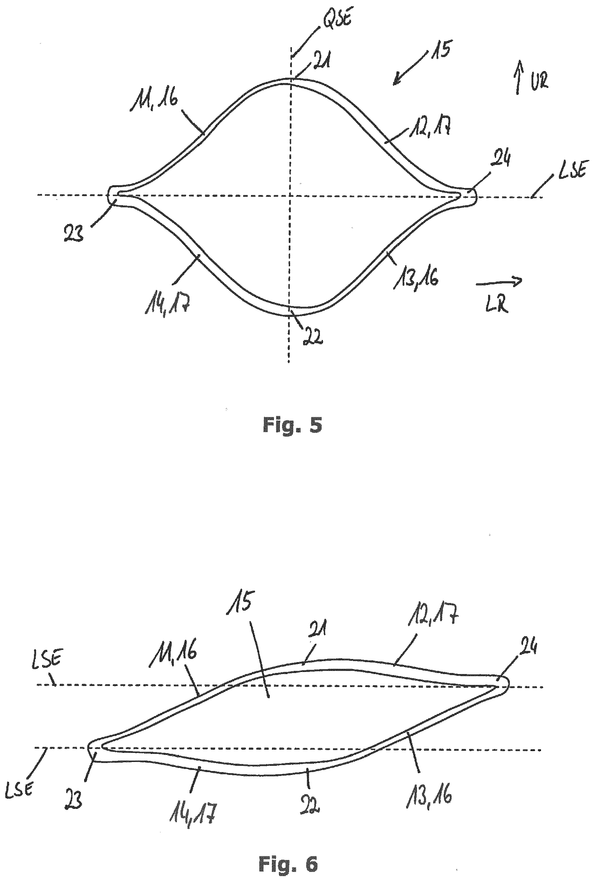

Die Torsion der Gitterstruktur 10 beruht konstruktiv auf der unterschiedlichen Gestaltung der Stege des ersten Stegpaares 16 und des zweiten Stegpaares 17. Bei dem Ausführungsbeispiel gemäß Figuren 1-4 wird beispielsweise durch die unterschiedliche Form des ersten und dritten Stegs 11, 13 gegenüber dem zweiten und vierten Steg 12, 14 die unterschiedliche Verformbarkeit der Stege 11, 13 des ersten Stegpaars 16 gegenüber den Stegen 12, 14 des zweiten Stegpaars 17 und in der Folge die Torsion der Gitterstruktur 10 bewirkt. Alternativ oder zusätzlich kann vorgesehen sein, die Verformbarkeit bzw. Biegsamkeit oder Flexibilität der einzelnen Stege 11, 12, 13, 14 durch eine Variation der Stegbreite bzw. allgemein der Stegdimensionen einzustellen. Eine derartige Variante ist bei dem Ausführungsbeispiel gemäß Figuren 5 und 6 verwirklicht. Figur 5 zeigt eine freigeschnittene, geschlossene Zelle 15, die durch Stege 11, 12, 13, 14 begrenzt ist. Die Stege 11, 12, 13, 14 sind analog zu dem Ausführungsbeispiel gemäß Figuren 1-4 an Verbindungsstellen 21, 22, 23, 24 miteinander verbunden. Die Zelle 15 weist im expandierten Zustand eine rautenförmige Grundform auf.

Die Stege 11, 12, 13, 14 der Zelle 15 gemäß Figur 5 weisen im Wesentlichen einen S-förmigen Verlauf zwischen zwei Verbindungsstellen 21, 22, 23, 24 auf. Die Form der Stege 11, 12, 13, 14 der Zelle 15 ist also im Wesentlichen gleich. Der erste Steg 11 und der dritte Steg 13, also die Stege 11, 13 des ersten Stegpaars 16, weisen jedoch eine Stegbreite auf, die kleiner als die Stegbreite des zweiten Stegs 12 und des vierten Stegs 14, also der ersten 12, 14 des zweiten Stegpaars 17, ist. Die Stegbreite des ersten und dritten Stegs 11, 13, also der Stege 11, 13 des ersten Stegpaars 16 untereinander ist gleich. Ebenso weisen der zweite Steg 12 und der vierte Steg 14, also die Stege 12, 14 des zweiten Stegpaars 17, die gleiche Stegbreite auf. Die Stege mit reduzierter Stegbreite, insbesondere der erste Steg 11 und der dritte Steg 13, sind daher verformbarer als die Stege 12, 14 des zweiten Stegpaares 17. Daraus resultiert bei der Zustandsänderung der Gitterstruktur 10 ein Versatz der dritten Verbindungsstelle 23 und der vierten Verbindungsstelle 24 in Umfangsrichtung UR der Gitterstruktur 10, wie in Figur 6 dargestellt. Insgesamt verdreht sich somit die Zelle 15. Da die Zelle 15 Teil eines Umfangssegments 20 ist, das aus gleichartig ausgebildeten Zellen 15 aufgebaut ist, erfolgt insgesamt eine Torsion der Gitterstruktur 10 bei einer Zustandsänderung, insbesondere einer Expansion oder Komprimierung der Gitterstruktur 10.

Der Versatz der in Längsrichtung gegenüberliegend angeordneten Verbindungsstellen 23, 24 der einzelnen Zellen 15 ist aufgrund der paarweisen Anordnung der Stege 11, 12, 13, 14 gleich. Das bedeutet, dass die dritte Verbindungsstelle 23 um den selben Betrag aus einer Ruheposition in Umfangsrichtung der Gitterstruktur 10 ausgelenkt wird, wie auch die vierte Verbindungsstelle 24. Der Betrag der Auslenkung ist also gleich, wobei die Richtung der Auslenkung jedoch unterschiedlich ist. Beispielsweise kann die dritte Verbindungsstelle 23 bei der Komprimierung der Gitterstruktur 10 in Uhrzeigerrichtung aus der Ruheposition ausgelenkt werden, wogegen die vierte Verbindungsstelle 24 in Gegenuhrzeigerrichtung aus der Ruhelage ausgelenkt wird.

Eine weitere Möglichkeit, die Verformbarkeit, Biegsamkeit oder Flexibilität der Stege 11, 12, 13, 14 der Gitterstruktur 10 unterschiedlich einzustellen, besteht darin, die Form und/oder die Dimension der Stege 11, 12, 13, 14 zumindest abschnittsweise zu ändern. Beispielsweise können Biegestellen 18 vorgesehen sein, die die Verformbarkeit einzelner Stege 11, 12, 13, 14 erhöhen. Die Biegestellen 18 können beispielsweise durch Verjüngungen gebildet sein, wobei die Stegbreite eines Stegs 11, 12, 13, 14 abschnittsweise reduziert ist. Alternativ oder zusätzlich kann in den Biegestellen 18 auch die Stegdicke eines Stegs 11, 12, 13, 14 bereichsweise reduziert sein.

Bei dem Ausführungsbeispiel gemäß Figur 7 ist vorgesehen, dass der erste Steg 11 und der dritte Steg 13, also die Stege 11, 13 des ersten Stegpaars 16, jeweils eine Biegestelle 18 umfassen. Die Biegestelle 18 bildet einen Abschnitt des jeweiligen Stegs 11, 13, in dem Stegbreite gegenüber der Stegbreite der Stege 12, 14 des zweiten Stegpaars 17 reduziert ist. Dadurch weisen der ersten Steg 11, und der dritte 13 insgesamt eine höhere Flexibilität als der zweite Steg 12 und der vierte Steg 14 auf. Bei einer Zustandsänderung der Gitterstruktur 10, also beispielsweise beim Übergang der Gitterstruktur 10 vom expandierten Zustand in den komprimierten Zustand, sind daher der erste Steg 11 und der dritte Steg 13 verformbarer als der zweite Steg 12 und der vierte Steg 14. Bei der Zustandsänderung der Gitterstruktur 10 verformen sich also die Stege 11, 13 des ersten Stegpaars 16 stärker als die Stege 12, 14 des zweiten Stegpaars 17. Insgesamt verformt sich somit die Zelle 15 und geht von einer rautenförmigen Grundform im expandierten Zustand, wie in Figur 7 dargestellt, in eine parallelogrammartige Grundform über, wobei die in Längsrichtung gegenüberliegend angeordneten Verbindungsstellen 23, 24 der Zelle 15 zueinander versetzt werden.

Die Gitterstruktur 10 ist vorzugsweise Teil eines Behandlungssystems, wobei die Gitterstruktur 10 ein proximales axiales Ende aufweist, das mit einem distalen Ende eines Führungsdrahts fest, insbesondere drehfest, verbunden ist. Beim Einsatz des Behandlungssystems wird durch den Führungsdraht somit das proximale Ende der Gitterstruktur 10 im Wesentlichen ortsfest gehalten, so dass die Stege 11, 12, 13, 14 der Gitterstruktur 10 bei der Expansion der Gitterstruktur 10 schraubenförmig in ein Blutgerinnsel 31 einschneiden können. Die Expansion der Gitterstruktur 10 erfolgt vorzugsweise selbsttätig. Die Gitterstruktur 10 ist vorzugsweise selbstexpandierend ausgebildet. Beispielsweise weist die Gitterstruktur 10 ein Formgedächtnismaterial, insbesondere eine Nickel-Titanlegierung auf, die die selbstexpandierenden Eigenschaften bewirkt.

Im Rahmen der Anmeldung wird als Rotationsgrad das Verhältnis zwischen der Rotation der einzelnen Umfangssegmente 20, also dem Versatz zwischen dem in Längsrichtung gegenüberliegend angeordneten Verbindungsstellen 23, 24 eines Umfangssegments 20, und der Durchmesseränderung bei der Expansion oder Komprimierung der Gitterstruktur 10 bezeichnet. Der Rotationsgrad wird pro Umfangssegment 20 bestimmt. Es ist möglich, dass sich der Rotationsgrad entlang der Gitterstruktur 10 ändert bzw. variiert wird. Dies kann beispielsweise dadurch erreicht werden, dass unterschiedliche Umfangssegmente 20 einen unterschiedlichen Rotationsgrad aufweisen. Der Rotationsgrad kann durch geeignete Dimensionierung der einzelnen Stegpaare eingestellt werden. Unterschiedliche Umfangssegmente können also verschiedenartig gestaltete Zellen 15 umfassen, wobei die Zellen 15 eines Umfangssegments 20 gleich sind. Die unterschiedlichen Umfangssegmente 20 können eine Änderung des Rotationsgrads entlang der Gitterstruktur 10 bewirken. Mit anderen Worten kann sich die Dynamik der Rotation bei einer Expansion der Gitterstruktur 10 ändern. Beispielsweise kann ein proximal angeordnetes Umfangssegment 20 bei der Expansion der Gitterstruktur 10 langsamer rotieren, also ein weiter distal angeordnetes Umfangssegment 20. Dabei ist es auch möglich, dass sich die Rotationsrichtung einzelner Umfangssegmente 20 unterscheidet. Die Rotationsrichtung der Gitterstruktur 10 kann sich also entlang der Gitterstruktur 10 ändern. Beispielsweise können einzelne Umfangssegmente in einem proximalen Bereich der Gitterstruktur 10 in Uhrzeigerrichtung rotieren, wogegen Umfangssegmente in einem distalen Bereich der Gitterstruktur 10 in Gegenuhrzeigerrichtung rotieren. In einem Extremfall, kann vorgesehen sein, dass ein mittlerer Abschnitt der Gitterstruktur 10 tordiert, wogegen die jeweiligen axialen Enden der Gitterstruktur 10 zueinander keine Relativbewegung in Umfangsrichtung der Gitterstruktur 10 ausführen. Auf jeden Fall wird die Rotation bzw. Torsion der Gitterstruktur 10 bereits allein durch die radiale Expansion oder Komprimierung ausgelöst.

Das Behandlungssystem kann auch mehr als eine Gitterstruktur 10 umfassen. Beispielweise können zwei Gitterstrukturen 10 überlagert werden, so dass sich bei der Expansion der beiden Gitterstrukturen 10 zwischen den Stegen der Gitterstrukturen 10 eine Scherbewegung einstellt. Mit anderen Worten können zwei oder mehr Gitterstrukturen 10 ineinander angeordnet sein.

Die erfindungsgemäße Vorrichtung und insbesondere das erfindungsgemäße Behandlungssystem eignen sich für unterschiedliche Einsatzzwecke. Beispielsweise können mit Hilfe der erfindungsgemäßen Vorrichtung Blutgerinnsel 31 oder Thromben von einer Gefäßwand getrennt bzw. abgeschält werden. Dabei wird die Gitterstruktur 10 bis zum Anschlag an die Gefäßwand expandiert und rotiert dabei zwischen der Gefäßwand und dem Blutgerinnsel 31. Die Expansion erfolgt dabei vorzugsweise durch Schieben der Gitterstruktur 10 aus einem Zuführkatheter, wobei der Zuführkatheter ortsfest gehalten wird. Das distale Ende der Gitterstruktur 10 kann insbesondere für diesen Einsatzzweck abgerundet sein, um atraumatisch zu wirken, also eine Gefäßverletzung zu vermeiden. Alternativ kann das distale Ende der Gitterstruktur 10 Schneidkanten aufweisen, die ein Abtrennen des Blutgerinnsels von der Gefäßwand begünstigen.

Ein weiterer Einsatzbereich der erfindungsgemäßen Vorrichtung bzw. des Behandlungssystems besteht in der Behandlung von Plaque. Plaque in Blutgefäßen können beispielsweise schichtweise abgetragen werden. Die Torsionsbewegung der Gitterstruktur 10 wirkt dabei ähnlich einem Fräser, der die Plaque schichtweise abträgt. Für diesen Einsatzzweck ist die Gitterstruktur 10 vorzugsweise vergleichsweise grobmaschig ausgebildet, weist also relativ große Zellöffnungen auf. Die Gitterstruktur 10 umfasst ferner vergleichsweise stabile Stege 11, 12, 13, 14, so dass die Gitterstruktur 10 eine hohe Radialkraft aufweist. Die Expansion der Gitterstruktur 10 zum Abtragen von Plaque erfolgt vorzugsweise dadurch, dass die Gitterstruktur 10 ortsfest gehalten und ein die Gitterstruktur 10 einhüllender Katheter in proximale Richtung abgezogen wird.

Ferner kann die erfindungsgemäße Vorrichtung oder das Behandlungssystem eingesetzt werden, um ein Blutgerinnsel zu zertrümmern. Dabei wird die Gitterstruktur 10 nicht vollständig bis zur Gefäßwand expandiert, sondern im Blutgerinnsel verankert. Insbesondere für den vorgenannten Einsatzzweck kann vorgesehen sein, dass die medizinische Vorrichtung innerhalb eines Schutzkorbes angeordnet ist, in dem das Blutgerinnsel eingekapselt wird. Ein derartiges Behandlungssystem, das einen Schutzkorb umfasst, in dem die medizinische Vorrichtung bzw. die Gitterstruktur 10 angeordnet ist, ist nicht auf diesen Einsatzzweck eingeschränkt.

Ferner kann die medizinische Vorrichtung, insbesondere im Zusammenhang mit dem Behandlungssystem, in Kombination mit einer Saugeinheit verwendet werden. Konkret das Behandlungssystem eine Saugeinheit umfassen, die mit der Gitterstruktur 10 bzw. dem durch die Gitterstruktur 10 aufgespannten Hohlraum bzw. Hohlkanal gekoppelt ist. Dabei kann das Absaugen von Partikeln durch die Saugeinheit beispielsweise innerhalb der Gitterstruktur 10 erfolgen. Dazu kann vorteilhaft vorgesehen sein, dass die Gitterstruktur 10 eine Beschichtung aufweist, so dass der durch die Saugeinheit aufgebrauchte Unterdruck im Wesentlichen ausschließlich auf das zu entfernende Blutgerinnsel wirkt und ein Absaugen von Blut aus einem Blutgefäß weitgehend vermieden wird. Die Beschichtung kann insbesondere derart ausgebildet sein, dass die Zellöffnungen der Zellen 15 fluiddicht bedeckt sind. Alternativ kann das Absaugen auch durch eine separate Vorrichtung erfolgen. Zusätzlich kann ein korbartiges Element eingesetzt werden, in das das entfernte Blutgerinnsel bzw. Partikel des entfernten Blutgerinnsels eingesaugt werden. Die erfindungsgemäße medizinische Vorrichtung bzw. die Gitterstruktur 10 der Vorrichtung kann in das Blutgerinnsel expandiert werden derart, dass das Blutgerinnsel zertrümmert wird. Die abgetrennten Partikel des Blutgerinnsels können anschließend über dies separate Saugeinrichtung entfernt werden.

Im Allgemeinen eignet sich die erfindungsgemäße Vorrichtung bzw. das erfindungsgemäße Behandlungssystem nicht nur zum Entfernen von Blutgerinnseln aus Blutgefäßen, sondern generell auch zum Entfernen von andersartigen Konkrementen aus Körperhohlorganen.

Die Gitterstruktur 10 ist vorzugsweise derart komprimierbar, dass sie in einem Zuführkatheter einführbar ist, der einen Innendurchmesser von weniger als 1,8 mm, insbesondere weniger als 1,4 mm, insbesondere weniger als 1,0 mm, insbesondere weniger als 0,72 mm, insbesondere weniger als 0,05 mm, insbesondere weniger als 0,42 mm, aufweist.

### Bezugszeichenliste

- 10: Gitterstruktur
- 11: erster Steg
- 12: zweiter Steg
- 13: dritter Steg
- 14: vierter Steg
- 15: Zelle
- 16: erstes Stegpaar
- 17: zweites Stegpaar
- 18: Biegestelle
- 20: Umfangssegment
- 21: erste Verbindungsstelle
- 22: zweite Verbindungsstelle
- 23: dritte Verbindungsstelle
- 24: vierte Verbindungsstelle
- 30: Blutgefäß
- 31: Blutgerinnsel
- LSE: Längsschnittebene
- QSE: Querschnittsebene
- LR: Längsrichtung
- UR: Umfangsrichtung

## Patentansprüche

1. Medizinische Vorrichtung mit einer komprimierbaren und expandierbaren, kreiszylinderförmigen Gitterstruktur (10), die Umfangssegmente (20) aus geschlossenen Zellen (15) umfasst, wobei die Zellen (15) durch jeweils vier Stege (11, 12, 13, 14) begrenzt sind, die an Verbindungsstellen (21, 22, 23, 24) miteinander gekoppelt sind und
von denen jeweils zwei gegenüberliegend angeordnete Stege (11, 13, 12, 14) gleichartig ausgebildet sind und ein Stegpaar (16) bilden, wobei die Stege (11, 13) eines ersten Stegpaares (16) zumindest abschnittsweise eine andere Form und/oder eine andere Stegbreite als die Stege (12, 14) eines zweiten Stegpaares (17) aufweisen derart, dass die Stege (11, 13) des ersten Stegpaares (16) beim Übergang der Gitterstruktur (10) vom expandierten Zustand in den komprimierten Zustand stärker verformbar als die Stege (12, 14) des zweiten Stegpaares (17) sind,
wobei jeweils ein Steg (11, 13) des ersten Stegpaares (16) mit einem Steg (12, 14) des zweiten Stegpaares (17) gekoppelt ist derart, dass zwei in Längsrichtung LR der Gitterstruktur (10) gegenüberliegend angeordnete Verbindungsstellen (23, 24) beim Übergang der Gitterstruktur (10) vom expandierten Zustand in den komprimierten Zustand in Umfangsrichtung UR der Gitterstruktur (10) gegenläufig zueinander versetzt werden,
wobei alle Zellen (15) eines Umfangssegments (20) gleichartig ausgebildet sind derart, dass die gesamte Gitterstruktur (10) beim Übergang vom expandierten Zustand in den komprimierten Zustand zumindest abschnittsweise tordiert.

2. Vorrichtung nach Anspruch 1,
**dadurch gekennzeichnet, dass**
jede Zelle (15) vier Verbindungsstellen (21, 22, 23, 24) umfasst, die im expandierten Zustand der Gitterstruktur (10) eine rautenförmige Grundform der Zelle (15) aufspannen.

3. Vorrichtung nach Anspruch 1 oder 2,
**dadurch gekennzeichnet, dass**
die Stege (11, 13) des ersten Stegpaares (16) im Wesentlichen S-förmig und die Stege (12, 14) des zweiten Stegpaares (17) im Wesentlichen geradlinig ausgebildet sind.

4. Vorrichtung nach einem der Ansprüche 1-3,
**dadurch gekennzeichnet, dass**
die Stege (11, 13) des ersten Stegpaares (16) eine Stegbreite aufweisen, die kleiner als die Stegbreite der Stege (12, 14) des zweiten Stegpaares (17) ist.

5. Vorrichtung nach einem der Ansprüche 1-4,
**dadurch gekennzeichnet, dass**
die Stege (11, 13) des ersten Stegpaares (16) jeweils wenigstens eine Biegestelle (18) aufweisen, an der die Stegbreite und/oder die Stegdicke des jeweiligen Stegs (11, 13) abschnittsweise reduziert oder erhöht ist.

6. Vorrichtung nach einem der Ansprüche 1-5,
**dadurch gekennzeichnet, dass**
die Stege (11, 12, 13, 14) an den Verbindungsstellen (21, 22, 23, 24) einstückig verbunden sind.

7. Vorrichtung nach einem der Ansprüche 1-6,
**dadurch gekennzeichnet, dass**
die Umfangssegmente (20) jeweils zwei Teilsegmente umfassen, die jeweils meanderförmig angeordnete Stege (11, 12, 13, 14) aufweisen, wobei jeder zweite Steg (11, 12, 13, 14) eines Teilsegments gleichartig ausgebildet ist.

8. Behandlungssystem mit einer medizinischen Vorrichtung gemäß einem der Ansprüche 1-7 und mit einem Katheter, in dem ein Führungselement längsverschieblich angeordnet ist, wobei das Führungselement fest, insbesondere drehfest, mit einem proximalen Ende der Gitterstruktur (10) der medizinischen Vorrichtung verbunden ist.

## Claims

1. Medical device having a compressible and expandable circular cylindrical lattice structure (10) that comprises circumferential segments (20) consisting of closed cells (15), wherein each of the cells (15) is delimited by four struts (11, 12, 13, 14) that are joined with each other at connection points (21, 22, 23, 24) and
of which the two struts (11, 13, 12, 14) arranged opposite each other are constructed similarly and constitute a strut pair (16), wherein at least sections of the struts (11, 13) of a first strut pair (16) have a different shape and/or strut width from the struts (12, 14) of a second strut pair (17), such that the struts (11, 13) of the first strut pair (16) are susceptible to greater deformation than the struts (12, 14) of a second strut pair (17) when the lattice structure (10) undergoes transition from the expanded state to the compressed state,
wherein one strut (11, 13) of each first strut pair (16) is joined to a strut (12, 14) of the second strut pair (17) in such manner that two connection points (23, 24) arranged opposite one another in the longitudinal direction LR of the lattice structure (10) are offset with respect to each other in opposite directions in the circumferential direction UR of the lattice structure (10) when the lattice structure (10) undergoes transition from the expanded state to the compressed state,
wherein all cells (15) of a circumferential segment (20) are formed similarly, such that at least sections of the entire lattice structure (10) undergo torsional deformation when said lattice structure undergoes transition from the expanded state to the compressed state.

2. Device according to claim 1,
**characterized in that**
each cell (15) has four connection points (21, 22, 23, 24) which enclose a rhomboid basic shape of the cell (15) when the lattice structure (10) is in the expanded state.

3. Device according to either of claims 1 or 2,
**characterized in that**
the struts (11, 13) of the first strut pair (16) are essentially S-shaped, and the struts (12, 14) of the second strut pair (17) are essentially straight.

4. Device according to any one of claims 1 to 3,
**characterized in that**
the struts (11, 13) of the first strut pair (16) have a strut width that is smaller than the struts (12, 14) of the second strut pair (17).

5. Device according to any one of claims 1 to 4,
**characterized in that**
the struts (11, 13) of the first strut pair (16) have at least one flexing point (18), where the strut width or strut thickness of the respective strut (11, 13) is reduced or increased in sections.

6. Device according to any one of claims 1 to 5,
**characterized in that**
the struts (11, 12, 13, 14) are connected integrally at the connection points (21, 22, 23, 24).

7. Device according to any one of claims 1 to 6,
**characterized in that**
the circumferential segments (20) each comprise two subsegments that have struts (11, 12, 13, 14) arranged in a meandering configuration, wherein every second strut (11, 12, 13, 14) of a subsegment is of similar construction.

8. Treatment system with a medical device according to any one of claims 1 to 7, and with a catheter in which a guide element is arranged so as to be longitudinally displaceable, wherein the guide element is connected in fixed manner, particularly in non-rotating manner, to a proximal end of the lattice structure (10) of the medical device.

## Revendications

1. Dispositif médical ayant une structure grillagée comprimable et expansible de forme cylindrique circulaire (10) qui comprend des segments circonférentiels (20) faits de cellules fermées (15), dans lequel les cellules (15) sont limitées respectivement par quatre traverses (11, 12, 13, 14) qui sont couplées entre elles à des points de liaison (21, 22, 23, 24) et
parmi lesquelles deux traverses disposées à l'opposé l'une de l'autre (11, 13, 12, 14) ont une conformation similaire et forment une paire de traverses (16), les traverses (11, 13) d'une première paire de traverses (16) présentant du moins par sections une autre forme et/ou une autre largeur de traverses que les traverses (12, 14) d'une seconde paire de traverses (17) de manière à ce que les traverses (11, 13) de la première paire de traverses (16), lors du passage de la structure grillagée (10) de l'état expansé à l'état comprimé, soient plus fortement déformables que les traverses (12, 14) de la seconde paire de traverses (17),
respectivement une traverse (11, 13) de la première paire de traverses (16) étant couplée à une traverse (12, 14) de la seconde paire de traverses (17) de manière à ce que deux points de liaison (23, 24) disposés à l'opposé l'un de l'autre dans le sens longitudinal LR de la structure grillagée (10), lors du passage de la structure grillagée (10) de l'état expansé à l'état comprimé, soient décalés l'un par rapport à l'autre en sens inverse dans le sens circonférentiel UR de la structure grillagée (10), toutes les cellules (15) d'un segment circonférentiel (20) ayant une conformation similaire de manière à ce que l'ensemble de la structure grillagée (10) se torde du moins par sections lors du passage de l'état expansé à l'état comprimé.

2. Dispositif selon la revendication 1,
**caractérisé en ce que**
chaque cellule (15) comprend quatre points de liaison (21, 22, 23, 24) qui, à l'état expansé de la structure grillagée (10), sous-tendent une forme de base en losange de la cellule (15).

3. Dispositif selon la revendication 1 ou 2,
**caractérisé en ce que**
les traverses (11, 13) de la première paire de traverses (16) sont réalisées sensiblement en forme de S et les traverses (12, 14) de la seconde paire de traverses (17) ont une conformation sensiblement rectiligne.

4. Dispositif selon une des revendications 1-3,
**caractérisé en ce que**
les traverses (11, 13) de la première paire de traverses (16) présentent une largeur de traverses qui est inférieure à la largeur de traverses les traverses (12, 14) de la seconde paire de traverses (17).

5. Dispositif selon une des revendications 1-4,
**caractérisé en ce que** les traverses (11, 13) de la première paire de traverses (16) présentent au moins un point de flexion (18) au niveau duquel la largeur de traverse et/ou l'épaisseur de traverse de la traverse respective (11, 13) diminue ou augmente par sections.

6. Dispositif selon une des revendications 1-5,
**caractérisé en ce que**
les traverses (11, 12, 13, 14) sont reliées en un seul bloc aux points de liaison (21, 22, 23, 24).

7. Dispositif selon une des revendications 1-6,
**caractérisé en ce que**
les segments circonférentiels (20) comprennent respectivement deux sous-segments qui présentent respectivement des traverses disposées serpentiformément (11, 12, 13, 14), une traverse sur deux (11, 12, 13, 14) d'un sous-segment ayant une conformation similaire.

8. Système de traitement comportant un dispositif médical selon une des revendications 1-7 et un cathéter, dans lequel un élément de guidage est disposé en mobilité longitudinale, l'élément de guidage étant relié fixement, en particulier fixement en rotation, à une extrémité proximale de la structure grillagée (10) du dispositif médical.
